# EUROPEAN PATENT APPLICATION

(11) **EP 4 193 929 A1**
(43) Date of publication of application: **14.06.2023**
(21) Application number: 21290082.3
(22) Date of filing: 09.12.2021
(51) Int. Cl.: A61B 8/08, G06T 7/00

(54) **ULTRASOUND IMAGING OF A FETUS**

(71) Applicant: Koninklijke Philips N.V., 5656 AG Eindhoven (NL)
(72) Inventor: Raynaud, Caroline Denise Francoise, 5656 AE Eindhoven (NL); Ciofolo, Cybèle, 5656 AE Eindhoven (NL); Olivier, Antoine, 5656 AE Eindhoven (NL); Germond Rouet, Laurence, 5656 AE Eindhoven (NL)
(74) Representative: Philips Intellectual Property & Standards

(57) **Abstract**

A mechanism for generating an estimated gestational age (GA). Ultrasound imaging data of a target fetus is obtained, and compared to a plurality of different models. Each model represents the structure of the brain/head of a fetus at a different gestational age. The model that most closely resembles the structure of the target fetus, as representing by the ultrasound imaging data, is identified. The associated gestational age of this model is used to generate the estimated gestational age of the subject.

## Description

### FIELD OF THE INVENTION

The present invention relates to the field of ultrasound imaging, and in particular, to the ultrasound imaging of a fetus.

### BACKGROUND OF THE INVENTION

During pregnancy of a subject, ultrasound imaging is a common technique used to assess the condition of the fetus. One useful parameter in assessing the fetus is an estimated gestational age (GA), which provides useful information for tracking and monitoring growth of the fetus and whether or not the fetus is under developing.

Various mechanisms for estimating the gestational age (GA) of a fetus are known. Typical approaches involve mapping one or more biometrical measurements to an estimated gestational age. Example biometric measurements include the fetus's femur length, head circumference and abdomen circumference. Estimations of the biometric measurements can be derived by directly measuring the fetus volume from 2D ultrasound acquisitions (i.e. from ultrasound imaging data).

There is an ongoing desire to improve the accuracy of estimates of gestational age and to provide additional information about the development of a fetus.

### SUMMARY OF THE INVENTION

The invention is defined by the claims.

According to examples in accordance with an aspect of the invention, there is provided a computer-implemented method of predicting a gestational age of a target fetus.

The computer-implemented method comprises: obtaining head ultrasound imaging data of the target fetus, containing a representation of the brain and/or head of the target fetus; obtaining a plurality of models, each model representing a structure of the brain and/or head of a fetus at a different gestational age; processing the head ultrasound imaging data and the plurality of models to identify the model, amongst the plurality of models, that most closely fits to the structure of the brain and/or head of the target fetus; and generating a first prediction of the gestational age of the target fetus using the gestational age associated with the identified model.

The present disclosure proposes an approach for estimating the gestational age (GA) of a fetus by comparing the structure of the brain/head of the fetus to models of the brain/head of a (generalized or average) fetus at different gestational ages. It has been recognized that the shape and development of (certain) structural features of the fetal brain reflect the gestational age of the fetus. Identifying a most closely fitting model to predict gestational age provides a more accurate and more reliable mechanism for predicting the gestational age, as it has less reliance upon specific measures, such as measurements of biological features which may be more prone to error. Moreover, this approach allows the evolution of different structural elements of the fetal brain over time to be used in the estimation of gestational age which means that there is not a reliance on any (single) specific structural element or feature of the fetal brain, which further increases reliability and accuracy of the gestational age estimation.

Embodiments therefore provide a mechanism for accurate and reliable prediction of gestational age, i.e. for generating an estimated gestational age.

The term "fetus" is here used to refer to unborn offspring of an animal (e.g. a mammal, reptile or bird). For the sake of the present disclosure, a fetus is considered to include any unborn offspring of an animal.

Preferably, the head ultrasound imaging data is 3D ultrasound imaging data, and each model is a 3D model. This provides a more accurate and reliable mechanism for identifying the model that most closely fits or represents the structure of the brain and/or head of the target fetus.

Optionally, the step of processing the head ultrasound imaging data and the plurality of models comprises: segmenting the ultrasound imaging data to generate a segmentation mask representing the structure of the brain and/or head of the target fetus; fitting the segmentation mask to each model of the plurality of models; and identifying the model that most closely or best fits the segmentation mask.

Generation of a segmentation mask produces suitably formatted data for comparison to the models. This approach thereby provides a mechanism for reliable generation of data that can be used to identify the model that most closely represents the structure of the brain and/or head of the target fetus.

In some examples, the step of processing the head ultrasound imaging data and the plurality of models comprises: segmenting the ultrasound imaging data to generate a segmentation mask representing the structure of the brain and/or head of the target fetus; comparing the segmentation mask and the part of the head ultrasound imaging data bound by the segmentation mask to each model of the plurality of models; and identifying the model that most closely or best matches the segmentation mask and the part of the head ultrasound imaging data bound by the segmentation mask.

The step of segmenting the ultrasound imaging data may comprise processing the ultrasound imaging data to detect the presence or absence of one or more structural elements of the brain and/or head of the target fetus; and segmenting the ultrasound imaging data to generate a segmentation mask that identifies the shape of only structural elements that are detected as present in the brain and/or head of the target fetus.

This approach provides a more efficient mechanism for segmenting the ultrasound imaging data, as only those structural elements that are known or predicted to be in the ultrasound imaging data are segmented (or are attempted to be segmented). Of course, the detection and segmentation tasks may be jointly performed, e.g. using a single machine-learning algorithm such as those that employ a Mask-RCNN architecture. Other architectures would also be suited to this task.

The step of segmenting the ultrasound imaging data may comprise segmenting the ultrasound imaging data using a neural network. A neural network provides suitable and reliable processing capability for segmenting ultrasound imaging data. Suitable neural networks include the U-net neural network and/or a convolutional neural network, such as a recursive convolutional neural network.

The computer-implemented method may further comprise obtaining body ultrasound imaging data, containing a representation of a portion of the body of the target fetus; processing the body ultrasound imaging data to determine one or more anatomical measurements of the target fetus; generating a second prediction of the gestational age of the target fetus by processing the determined anatomical measurements; and generating a small for gestational age, SGA, indicator responsive to a difference between the first prediction and the second prediction, wherein the SGA indicator indicates a prediction of whether or not the target fetus is small for its gestational age.

An SGA indicator provides valuable information for a clinician or caregiver on the progress and development of the fetus. In particular, an SGA indicator is a useful clinical data point for assessing whether medical intervention is needed, either during pregnancy and/or after birth, as well as a strong indicator of pregnancy risk. An SGA fetus (i.e. a fetus that is small for its gestational age) has increased risk of medical problems, and an early marker or indicator of SGA is useful for making clinical decisions.

It has been recognized that, although brain development of a fetus is affected by fetal growth restriction, the spatial arrangement of many cerebral structures and their relative size is not significantly different on normal fetuses from those measured on SGA fetuses. In this way, a discrepancy or difference between the first prediction (based on the structure of the brain) and the second prediction (based on body imaging data) represents or indicates a possible SGA fetus.

The second prediction may be generated by estimating the one or more biological measurements of the fetus from the imaging data, and converting these measurements into a gestational age using standard development charts. Examples of suitable biological measurements include the head circumference, abdomen circumference, femur length and/or a global volume, of the fetus. Any of these values may be identified, for instance, by segmenting the body ultrasound imaging data, e.g. using an appropriately trained machine-learning algorithm and/or contouring mechanisms. If multiple biological measurements are identified, the second prediction may be generated by combining the converted gestational age of each biological measurement (e.g. using an averaging, weighted averaging or majority vote approach).

The body ultrasound imaging data may comprise two-dimension (2D) or three-dimensional (3D) ultrasound data of the subject. Processing the ultrasound imaging data may comprise determining measurements and/or biometry data of the portion of the body of the target fetus, e.g. identify precise measurements of anatomical features and/or elements of the target fetus. This information is usable to derive or estimate a gestational age from the body ultrasound imaging data using conventional prediction techniques.

The method may further comprise a step of providing a user-perceptible alert responsive to the SGA indicator meeting at least one predetermined criterion. This provides a clinician with useful clinical information that can be used to more accurately assess or understand the condition of the fetus, in order to aid the clinician in making a clinical decision.

The at least one predetermined criterion may comprise the SGA indicator indicating a prediction that the target fetus is small for its gestational age. The SGA indicator may indicate a prediction that the target fetus is small for its gestational age responsive to the difference between the first and second predictions exceeding a predetermined value. This embodiment recognizes that the size of the difference between the first and second predictions is a key indicator of whether the fetus is SGA. The greater the difference between the first and second predictions, the more likely that the fetus is SGA.

In some examples, the method further comprises determining a combined estimation of the gestational age of the target fetus by processing the first prediction and the second prediction.

In some embodiments, the method further comprises controlling a user-perceptible output responsive to the first prediction. This embodiment may comprise, for instance, controlling the user-perceptible output responsive to the first prediction alone, the SGA indicator alone (if generated) or the combined prediction alone (if generated).

The structure of the brain and/or head of the target fetus may include the shape of one or more cerebral structures, such as sulci. Even after the formation of the main cerebral structures, some morphological changes, such as the appearance of sulci, occur throughout. This is observed for many sulci and other cerebral structures. Accordingly, it has been recognized that the shape and/or appearance of such cerebral structures provides a good indicator of gestational age. Examples of suitable cerebral structures (that change shape throughout pregnancy) include the Sylvian fissure, the parieto-occipital fissure, the cingulate sulcus and the calcarine sulcus.

The present embodiment makes use of these different structures in order to provide models that accurately represent the evolution of the structure of the brain over the course of the pregnancy.

In preferred examples, the interval between consecutive gestational ages represented by the plurality of models is between one and two weeks. This embodiment recognizes that minor changes or deviations from an average pregnancy development are normal and not unexpected. By restricting the predicted gestational age to fall within certain discrete periods of time, these minor changes can be taken into account.

There is also proposed a computer program product comprising computer program code means which, when executed on a computing device having a processing system, cause the processing system to perform all of the steps of any herein described method.

There is also proposed a processing system configured to predict a gestational age of a target fetus, wherein the processing system is configured to: obtain head ultrasound imaging data of the target fetus, containing a representation of the brain and/or head of the target fetus; obtain a plurality of models, each model representing a structure of the brain and/or head of a fetus at a different gestational age; process the head ultrasound imaging data and the plurality of models to identify the model, amongst the plurality of models, that most closely fits to the structure of the brain and/or head of the target fetus; and generate a first prediction of the gestational age of the target fetus using the gestational age associated with the identified model.

The processing system may be adapted to perform any herein described method, and vice versa.

There is also proposed a system comprising: the processing system previously described and a user interface configured to provide a user-perceptible output responsive to the first prediction.

These and other aspects of the invention will be apparent from and elucidated with reference to the embodiment(s) described hereinafter.

### BRIEF DESCRIPTION OF THE DRAWINGS

For a better understanding of the invention, and to show more clearly how it may be carried into effect, reference will now be made, by way of example only, to the accompanying drawings, in which:
Fig. 1 is a flowchart illustrating a method;
Fig. 2 illustrates a change in the Sylvian fissure over the course of gestation;
Fig. 3 is a flowchart illustrating another method;
Fig. 4 illustrates a processing system; and
Fig. 5 illustrates a system.

### DETAILED DESCRIPTION OF THE EMBODIMENTS

The invention will be described with reference to the Figs.

It should be understood that the detailed description and specific examples, while indicating exemplary embodiments of the apparatus, systems and methods, are intended for purposes of illustration only and are not intended to limit the scope of the invention. These and other features, aspects, and advantages of the apparatus, systems and methods of the present invention will become better understood from the following description, appended claims, and accompanying drawings. It should be understood that the Figs. are merely schematic and are not drawn to scale. It should also be understood that the same reference numerals are used throughout the Figs. to indicate the same or similar parts.

The invention provides a mechanism for generating an estimated gestational age (GA). Ultrasound imaging data of a target fetus is obtained, and compared to a plurality of different models. Each model represents the structure of the brain/head of a fetus at a different gestational age. The model that most closely resembles the structure of the target fetus, as representing by the ultrasound imaging data, is identified. The associated gestational age of this model is used to generate the estimated gestational age of the subject.

Embodiments of the invention are based on the realization that a longitudinal set of models allows changes in key features of the fetal/head brain to be taken into account when attempting to predict the gestational age of the fetus. Use of models in this way provide a more accurate approach for estimating gestational age, as there is a reduced reliance upon accurate identification of certain measurements, as the approach is less sensitive to changes in precise measurements of anatomical features of the fetus.

Approaches proposed by the present disclosure may be used in any suitable clinical environment in which ultrasound data of a target fetus is available or can be obtained, e.g. during prenatal ultrasound scans.

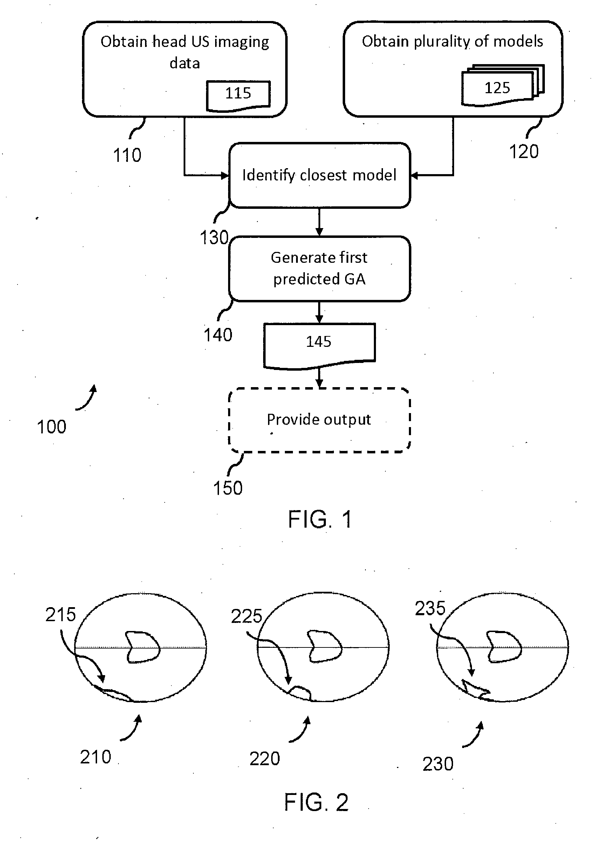

Fig. 1 illustrates a method 100 according to an embodiment of the invention. The method 100 is computer-implemented, i.e. can be performed by a computer or processing system.

The method 100 comprises a step 110 of obtaining head ultrasound imaging data 115 of the target fetus, containing a representation of the brain and/or head of the target fetus. The head ultrasound imaging data may be two-dimensional imaging data or three-dimensional imaging data. The head ultrasound imaging data may be obtained from a memory and/or directly from an ultrasound imaging system (e.g. actively scanning a target fetus).

The method 100 also comprises a step 120 of obtaining a plurality of models 125. The plurality of models each represents a structure of the brain and/or head of a fetus at a different gestational age. In particular, each model represents the (expected or average) shape of one or more features of the brain and/or head of the fetus at a particular gestational age. For instance, each model may be a mesh or other data structure that defines or represents a shape of the one or more features of the brain and/or head.

Thus, the plurality of models effectively forms a temporal sequence of models, each model representing an average or expected structure of the brain and/or head of a fetus at a particular gestational age. The temporal spacing between each model may be predetermined, e.g. 1 week or 2 weeks apart.

In particularly preferable examples, the interval between consecutive gestational ages represented by the plurality of models is between one and two weeks. This recognizes that minor changes or deviations from an average pregnancy development are normal and not unexpected (i.e. should not be a cause for concern). By restricting the predicted gestational age to fall within certain discrete periods of time, these minor changes can be taken into account. In particular, it may not be possible to be more precise about an estimated gestational age, due to natural and expected deviations in development time, such that memory and processing power can be saved by limiting the number of models to have intervals of only 1-2 weeks (as any further models will not increase an accuracy of the estimated gestational age):
The plurality of models thereby forms a longitudinal collection of models at different stages of pregnancy.

A property of fetal brain/head development exploited by the present invention is that the structure of the brain and/or head of a fetus continues to develop throughout the course of pregnancy. In particular, different parts of the structure of the brain and/or head develop and/or undergo morphological change throughout the course of pregnancy.

For instance, during the first trimester of pregnancy, cerebral structures of the fetus will develop and appear, so that the presence of particular cerebral structures may indicate the gestational age of the fetus.

Even after the formation of the main cerebral structures, some morphological' changes, such as the appearance of sulci, occur during the second and third trimester of pregnancy. This is observed for many sulci (such as the parieto-occipital fissure, the cingulate sulcus and the calcarine sulcus) but a particularly obvious example is the Sylvian fissure, whose shape evolves during a large part of the pregnancy.

Fig. 2 illustrates the evolution of the Sylvian fissure over the course of gestation or pregnancy. Each structural illustration may represent a different model of the head and/or body of a fetus, e.g. for use in the plurality of models.

During a first time period, represented by a first structural illustration 210, the Sylvian fissure 215 exhibits a smooth or no angle. Conventionally, this is associated with a time period of less than 18 weeks of gestational age.

During a second time period, represented by a second structural illustration 220, the Sylvian fissure 225 exhibits an obtuse angle. Conventionally, this is associated with a time period of between 17 and 25 weeks of gestational age.

During a third time period, represented by a third structural illustration 230, the Sylvian fissure 235 exhibits an acute angle. Conventionally, this is associated with a time period of more than 23 weeks of gestational age.

This example demonstrates how the shape, size and structure of a particular part of the brain/head of the fetus can indicate a particular gestational age, such that it is possible to build a collection of different models representing the expected structure of the head/brain of a fetus at different gestational ages.

More specific steps of the evolution of the Sylvian fissure over gestation, which could be exploited in the present invention, are disclosed by Toi A, L. W. (2004), How early are fetal cerebral sulci visible at prenatal ultrasound and what is the normal pattern of early fetal sulcal development, Ultrasound in Obstetrics and Gynecology, 706-715. Many other cerebral structures, located in various parts of the brain, also change shape and/or size, and could be used to similar effect.

In particular, by combining the evolution of several cerebral structures which do not appear or change at the same time (such as the Sylvian fissure, the parieto-occipital fissure, the cingulate sulcus and the calcarine sulcus), the collection of models is able to represent expected changes or structures of brain development over the course of gestational age, such that each model is distinguishable from the others.

Turning back to Fig. 1, the method 100 further comprises a step 130 of processing the head ultrasound imaging data and the plurality of models to identify the model, amongst the plurality of models, that most closely fits to the structure of the brain and/or head of the target fetus.

In this way, step 130 effectively, for a given ultrasound volume/section of the fetal brain, fits the models (of the longitudinal collection) to said ultrasound volume/section to identify the one that fits the best.

Various approaches can be used to perform step 130, and these approaches could be combined.

In a first example, a structure-detection technique is used to identify the presence and/or shape of structures contained within the ultrasound imaging data. For instance, a machine-learning method (such as a "you-only-look-once", YOLO, neural network) may be used to produce detection scores for a plurality of different structures. The machine-learning method may also be able to identify an approximate location of any identified structures (e.g. using bounding boxes or the like). The detection scores can then be used to assess the presence or absence of specific cerebral structures. This information may then be used to identify any models that match the specific cerebral structures that are present, to thereby identify the model that most closely represents the current state of the fetal brain/head.

The YOLO neural network is set out and explained by J Redmon, S. D. (2016). You Only Look Once: Unified, real-time object detection. CVPR, and could be readily adapted for use in embodiments of the present disclosure.

This first example is particular useful for identifying the closest model during the early stages (e.g. the first trimester) of pregnancy, in which the presence or absence of a particular cerebral structure is a strong indicator of gestational age.

In a second example, a segmentation technique is used to segment or identify shapes of cerebral structures of the brain.

Thus, step 130 may comprise segmenting the ultrasound imaging data to generate a segmentation mask representing the structure of the brain and/or head of the target fetus; fitting the segmentation mask to each model of the plurality of models; and identifying the model that most closely or best fits the segmentation mask.

In another example, step 130 comprises segmenting the ultrasound imaging data to generate a segmentation mask representing the structure of the brain and/or head of the target fetus; comparing the segmentation mask and the part of the head ultrasound imaging data bound by the segmentation mask to each model of the plurality of models; and identifying the model that most closely or best matches the segmentation mask and the part of the head ultrasound imaging data bound by the segmentation mask.

In this example, both the segmentation mask and parts of the ultrasound data included in the segmentation mask are used to identify the closest model to the ground truth of the head ultrasound imaging data. The additional use of the ultrasound data bound by the segmentation mask improves the fitting.

In some scenarios, a structure-detection technique (such as those previously described) is initially used to identify which cerebral structures are present (and preferably, the approximate location of the same), and one or more segmentation techniques are used to identify the shape of any identified cerebral structures. In some examples, each cerebral structure is associated with its own segmentation algorithm, specifically designed or trained for identifying a specific cerebral structure. In some scenarios, any suitable segmentation technique may be used such as the U-net technique disclosed by O Ronneberger, P. F. (2015). U-Net: Convolutional networks for biometical image segmentation. MICCAI, (pp. 234--241). Other segmentation techniques will be apparent to the skilled person, e.g. thresholding segmentation techniques, edge-based segementation techniques and so on.

In this way, segmenting the ultrasound image may comprise processing the ultrasound imaging data to detect the presence or absence of one or more structural elements of the brain and/or head of the target fetus; and segmenting the ultrasound imaging data to generate a segmentation mask that identifies the shape of only structural elements that are detected as present in the brain and/or head of the target fetus.

In other scenarios, the detection and segmentation tasks may be performed by a single process or architectures, for instance, using a mask-based segmentation techniques such as the Mask-RCNN architecture proposed in He K., Gkioxari G., Dollar P., Girshick R. "Mask R-CNN", 2017 IEEE International Conference on Computer Vision (ICCV). 2017. Other architectures may also be well-suited for this task.

In this way, the step of segmenting the ultrasound imaging data may comprise segmenting the ultrasound imaging data using a neural network.

Segmentation will produce segmentation masks whose shapes can be compared to those contained in the models. Thus, step 130 can be performed by fitting segmentation masks to each of the plurality of models to identify the best fitting result.

A best fitting result may, for instance, be identified by determining a measure of overlap between the shape of the masks and the shape of the models and/or a distance between contours of the mask and the models.

One approach for comparing a segmentation mask to a model may be to treat the segmentation mask as a model itself, and use model comparison techniques to determine a measure of similarity. The model with the greatest measure of similarity can be identified as the best fitting result.

Approaches for determining a similarity between models are known, such as those disclosed by Chen, Xin, et al. "Research on similarity measurements of 3d models based on skeleton trees." Computers 6.2 (2017): 17 or by Tangelder, Johan WH, and Remco C. Veltkamp. "Polyhedral model retrieval using weighted point sets." International journal of image and graphics 3.01 (2003): 209-229.

Thus, step 130 may comprise segmenting the ultrasound imaging data to generate a segmentation mask representing the structure of the brain and/or head of the target fetus; fitting the segmentation mask to each model of the plurality of models; and identifying the model that most closely or best fits the segmentation mask.

In yet another example, step 130 may comprise, for each model of the plurality of models, performing a model-based segmentation (e.g. using deformable meshes) of the ultrasound imaging data using the model (e.g. to iteratively modify the model to fit to the ultrasound imaging data). The number of iterations required to adapt a model to the ultrasound imaging data is inversely proportional to the similarity between the model and the structure illustrated in the ultrasound imaging data. Thus, a measure of similarity can be derived from performing model-based segmentation.

Suitable model-based segmentation techniques are well known in the art, such as those described by Tobias Hermann, Hervé Delingette. Model-based segmentation. Desemo, Thomas Martin. Biomedical Image Processing, Springer, pp.279-303, 2011.

In yet other examples, both the segmentation mask and part of the ultrasound data bound by the segmentation mask are used to identify the closest model.

After step 130, the method performs a step 140 of generating a first prediction 145 of the gestational age of the target fetus using the gestational age associated with the identified model.

Each model is associated with a particular gestational age. Generating the first prediction may thereby simply comprise identifying the gestational age associated with the identified model (i.e. the closest fitting model).

In a further embodiment, step 130 may comprise generating an indicator of closeness of fit for each of the plurality of models. Step 140 may comprise using the indicators of closeness of fit to generate the first prediction of gestational age. The indicator may, for instance, be a numeric measure of closeness of fit.

In a simple example, step 140 may comprise simply identifying the gestational age associated with the model having the indicator that indicates a closest measure of fit.

In another example, e.g. to be used if the indicators of closeness of fit for ultrasound imaging data is similar for two models of the plurality of models, then step 140 may comprise setting the first prediction of gestational age to fall (e.g. proportionally) between the gestational age associated with the two models.

As yet another example, the gestational age of all models having indicators of closeness of fit that meets some predetermined criterion/criteria may be used (e.g. averaged) to generate the first prediction. For instance, if the indicator is a numeric measure of closeness of fit on a predetermined scale, the gestational age of all models whose indicator exceeds some predetermined value may be averaged to generate the first prediction of gestational age.

The method 100 may further comprise a step 150 of controlling a user-perceptible output responsive to the first prediction. The user-perceptible output may be a visual, audio and/or haptic output that is detectable by an individual. In particular, a visual representation of the first prediction may be provided at a display or user interface. In some examples, an audio representation (e.g. using voice/speech synthesis) of the first prediction may be provided by a speaker or other audio output device.

Of course, step 150 may be omitted, as the first prediction may be used for other processes or stored for later analysis. For instance, a predicted GA may be useful in identifying one or more pathologies of the fetus, and could be used as an input to an analysis or assessment methodology, e.g. an input to a machine-learning method for analyzing the fetus.

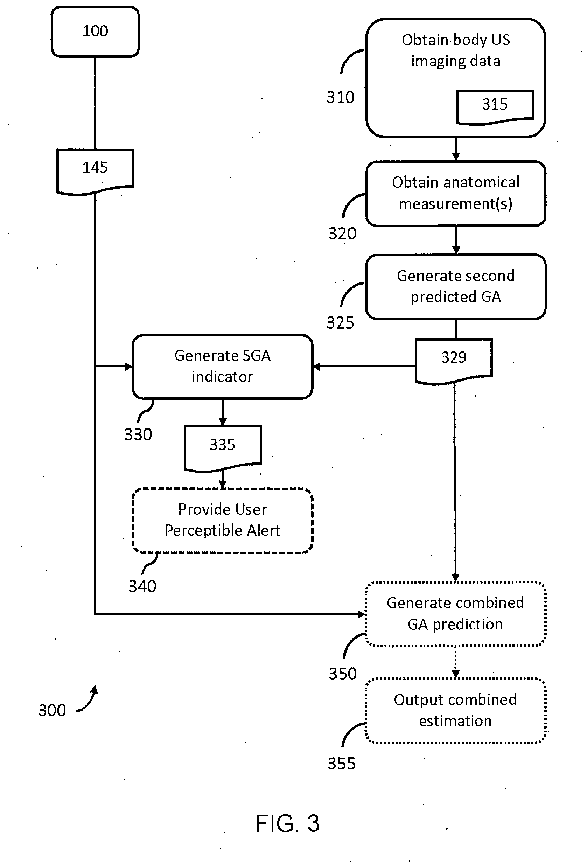

Fig. 3 illustrates a method 300 according to another embodiment of the invention.

The method 300 comprises a process 100 of generating a first prediction 145 of the gestational age of the target fetus using a previously described approach (e.g. with reference to Fig. 1).

The method 300 further comprises a step 310 of obtaining body ultrasound imaging data 315, containing a representation of a portion of the body of the target fetus. The body ultrasound imaging data may comprise two-dimension (2D) or three-dimensional (3D) ultrasound data of the subject.

The method 300 comprises a step 320 of processing the body ultrasound imaging data to generate one or more anatomical measurements and then a step 325 of generating a second prediction 329 of the gestational age of the target fetus.

Techniques for estimating gestational age from body ultrasound imaging data are well established in the art. Typically, such techniques include determining one or more measurements of the fetus' anatomy (e.g. head circumference, abdomen circumference, femur length and fetal volume), and converting the determined measurement(s) into a predicted gestational age. The conversion may make use of known or standard development charts, such as those presented in A T Papageorghiou, E. O. (2014). International standards for fetal growth based on serial ultrasound measurements: the Fetal Growth Longitudinal Study of the INTERGROWTH-21st project. Lancet, 869--879. In this way, a simple look-function can be used to generate the second prediction of gestational age from measurements of the fetus' anatomy.

Approaches for determining one or more measurements from body ultrasound imaging data will be readily apparent to the skilled person. Some approaches may use a segmentation technique to segment anatomical features, and use automated calipers or the like to determine anatomical methods. Any suitable segmentation technique could be adopted for this purpose, such as a machine-learning method, e.g. a neural network.

It will be appreciated that some of the previously identified measurements (e.g. fetal volume) may only be derivable if the ultrasound imaging data is three-dimensional or contains a sufficient number of two-dimensional slices. Of course, if the ultrasound imaging data comprises only 2D imaging data, standard biometry measurements in 2D could be used to produce an estimation of the gestational age.

Steps 320 and 325 are distinguished from process 100 in that process 100 makes use of a plurality of models of a fetus' head and/or body to predict a gestational age. By contrast, steps 320 and 325 makes use of one or more anatomical measurements in order to predict the gestational age.

The method 300 then performs a step 330 of generating a small for gestational age, SGA, indicator 335 responsive to a difference between the first prediction 145 (generated by process 100) and the second prediction 329 (generated in step 325). The SGA indicator indicates a prediction of whether or not the target fetus is small for its gestational age.

This step recognizes that if the estimations produced by the cerebral structure development model approach (i.e. process 100) and the assessment of anatomical measurements, such as the fetal volume, approach (i.e. step 320) are consistent, a high confidence level can be associated to the estimated GA. If the estimations are different, especially if the GA estimated from the cerebral structure development model is significantly higher than the one from the anatomical measurement(s), there is a risk that the fetal growth is too low with respect to the brain development.

In particular, it has been observed that accelerated functional and anatomical brain development occur in fetuses suffering from growth restriction or exposed to maternal hypertension, which means that the discrepancy of GA estimated values is higher in such cases.

This means that it is possible to generate an indicator of small for gestational age (SGA indicator) responsive to the difference between the first and second estimations. In particular, if this difference exceeds a predetermined threshold value or percentage (e.g. percentage of one of the estimations), then the SGA indicator indicates that the target fetus is small for its gestational age. The predetermined threshold value may, for instance, be a week or two weeks.

In some preferred embodiments, the SGA indicator indicates the target fetus is small for its gestational age only if difference (which is here calculated by subtracting the second estimation from the first estimation) exceeds a predetermined threshold value or percentage. This embodiment recognizes that advanced cerebral development compared to anatomical size is indicative of SGA, e.g. that the fetus suffers from growth restrictions there is maternal hypertension. Such an indicator thereby provides more accurate information for alerting a clinician as to the condition of the fetus.

In some examples, the method 300 further comprises a step 340 of providing a user-perceptible alert responsive to the SGA indicator meeting at least one predetermined criterion. The at least one predetermined criterion comprises the SGA indicator indicating a prediction that the target fetus is small for its gestational age.

The user-perceptible alert may be a visual, audio and/or haptic alert that is detectable by an individual. In particular, a visual representation of the alert may be provided at a display or user interface. In some examples, an audio representation (e.g. using an alarm) of the alert may be provided by a speaker or other audio output device.

In some examples, the method 300 comprises a step 350 of determining a combined estimation of the gestational age of the target fetus by processing the first prediction and the second prediction. Step 350 may, for instance, comprise averaging the first prediction and the second prediction.

Of course, the method 300 may comprise a step 355 of controlling a user-perceptible output responsive to the combined estimation. The user-perceptible output may be a visual, audio and/or haptic alert that is detectable by an individual. In some examples, step 355 is performed instead of step 150 previously described.

The skilled person would be readily capable of developing a processing system for carrying out any herein described method. Thus, each step of the flow chart may represent a different action performed by a processing system, and may be performed by a respective module of the processing system.

Embodiments may therefore make use of a processing system. The processing system can be implemented in numerous ways, with software and/or hardware, to perform the various functions required. A processor is one example of a processing system which employs one or more microprocessors that may be programmed using software (e.g., microcode) to perform the required functions. A processing system may however be implemented with or without employing a processor, and also may be implemented as a combination of dedicated hardware to perform some functions and a processor (e.g., one or more programmed microprocessors and associated circuitry) to perform other functions.

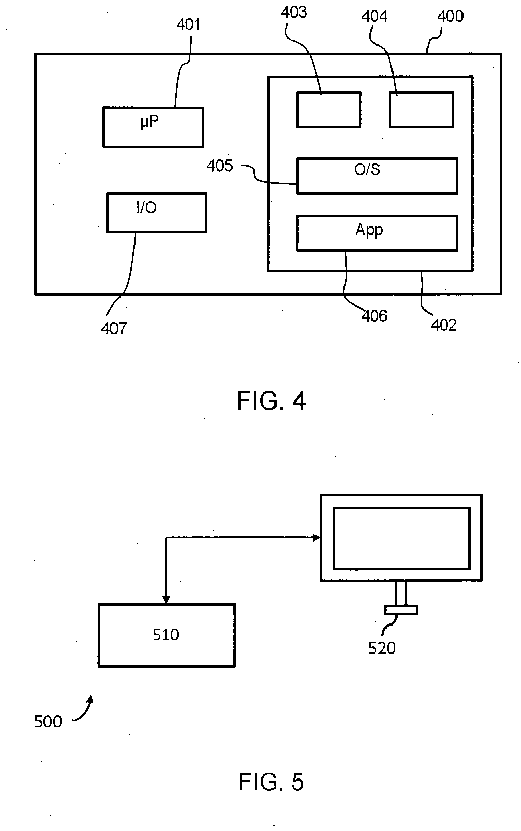

By way of further example, Fig. 4 illustrates an example of a computer 400 within which one or more parts of an embodiment may be employed. Various operations discussed above may utilize the capabilities of the computer 400. For example, one or more parts of a system for generating an estimation of gestational age and/or an SGA indicator may be incorporated in any element, module, application, and/or component discussed herein. In this regard, it is to be understood that system functional blocks can run on a single computer or may be distributed over several computers and locations (e.g. connected via internet).

The computer 400 includes, but is not limited to, PCs, workstations, laptops, PDAs, palm devices, servers, storages, and the like. Generally, in terms of hardware architecture, the computer 400 may include one or more processors 401, memory 402, and one or more I/O devices 407 that are communicatively coupled via a local interface (not shown). The local interface can be, for example but not limited to, one or more buses or other wired or wireless connections, as is known in the art. The local interface may have additional elements, such as controllers, buffers (caches), drivers, repeaters, and receivers, to enable communications. Further, the local interface may include address, control, and/or data connections to enable appropriate communications among the aforementioned components.

The processor 401 is a hardware device for executing software that can be stored in the memory 402. The processor 401 can be virtually any custom made or commercially available processor, a central processing unit (CPU), a digital signal processor (DSP), or an auxiliary processor among several processors associated with the computer 400, and the processor 401 may be a semiconductor based microprocessor (in the form of a microchip) or a microprocessor.

The memory 402 can include any one or combination of volatile memory elements (e.g., random access memory (RAM), such as dynamic random access memory (DRAM), static random access memory (SRAM), etc.) and non-volatile memory elements (e.g., ROM, erasable programmable read only memory (EPROM), electronically erasable programmable read only memory (EEPROM), programmable read only memory (PROM), tape, compact disc read only memory (CD-ROM), disk, diskette, cartridge, cassette or the like, etc.). Moreover, the memory 402 may incorporate electronic, magnetic, optical, and/or other types of storage media. Note that the memory 402 can have a distributed architecture, where various components are situated remote from one another, but can be accessed by the processor 401.

The software in the memory 402 may include one or more separate programs, each of which comprises an ordered listing of executable instructions for implementing logical functions. The software in the memory 402 includes a suitable operating system (O/S) 405, compiler 404, source code 403, and one or more applications 406 in accordance with exemplary embodiments. As illustrated, the application 406 comprises numerous functional components for implementing the features and operations of the exemplary embodiments. The application 406 of the computer 400 may represent various applications, computational units, logic, functional units, processes, operations, virtual entities, and/or modules in accordance with exemplary embodiments, but the application 406 is not meant to be a limitation.

The operating system 405 controls the execution of other computer programs, and provides scheduling, input-output control, file and data management, memory management, and communication control and related services. It is contemplated by the inventors that the application 406 for implementing exemplary embodiments may be applicable on all commercially available operating systems.

Application 406 may be a source program, executable program (object code), script, or any other entity comprising a set of instructions to be performed. When a source program, then the program is usually translated via a compiler (such as the compiler 404), assembler, interpreter, or the like, which may or may not be included within the memory 402, so as to operate properly in connection with the O/S 405. Furthermore, the application 406 can be written as an object oriented programming language, which has classes of data and methods, or a procedure programming language, which has routines, subroutines, and/or functions, for example but not limited to, C, C++, C#, Pascal, BASIC, API calls, HTML, XHTML, XML, ASP scripts, JavaScript, FORTRAN, COBOL, Perl, Java, ADA, .NET, and the like.

The I/O devices 407 may include input devices such as, for example but not limited to, a mouse, keyboard, scanner, microphone, camera, etc. Furthermore, the I/O devices 407 may also include output devices, for example but not limited to a printer, display, etc. Finally, the I/O devices 407 may further include devices that communicate both inputs and outputs, for instance but not limited to, a NIC or modulator/demodulator (for accessing remote devices, other files, devices, systems, or a network), a radio frequency (RF) or other transceiver, a telephonic interface, a bridge, a router, etc. The I/O devices 407 also include components for communicating over various networks, such as the Internet or intranet.

If the computer 400 is a PC, workstation, intelligent device or the like, the software in the memory 402 may further include a basic input output system (BIOS) (omitted for simplicity). The BIOS is a set of essential software routines that initialize and test hardware at startup, start the O/S 405, and support the transfer of data among the hardware devices. The BIOS is stored in some type of read-only-memory, such as ROM, PROM, EPROM, EEPROM or the like, so that the BIOS can be executed when the computer 400 is activated.

When the computer 400 is in operation, the processor 401 is configured to execute software stored within the memory 402, to communicate data to and from the memory 402, and to generally control operations of the computer 400 pursuant to the software. The application 406 and the O/S 405 are read, in whole or in part, by the processor 401, perhaps buffered within the processor 401, and then executed.

When the application 406 is implemented in software it should be noted that the application 406 can be stored on virtually any computer readable medium for use by or in connection with any computer related system or method. In the context of this document, a computer readable medium may be an electronic, magnetic, optical, or other physical device or means that can contain or store a computer program for use by or in connection with a computer related system or method.

The application 406 can be embodied in any computer-readable medium for use by or in connection with an instruction execution system, apparatus, or device, such as a computer-based system, processor-containing system, or other system that can fetch the instructions from the instruction execution system, apparatus, or device and execute the instructions. In the context of this document, a "computer-readable medium" can be any means that can store, communicate, propagate, or transport the program for use by or in connection with the instruction execution system, apparatus, or device. The computer readable medium can be, for example but not limited to, an electronic, magnetic, optical, electromagnetic, infrared, or semiconductor system, apparatus, device, or propagation medium.

Fig. 5 illustrates a system 500 according to an embodiment.

The system 500 comprises a processing system 510, which is itself an embodiment of the invention. The processing system 510 may be embodied as previously described with reference to Fig. 4, although other forms of processing system will be apparent to the skilled person. The processing system 510 is configured to perform any herein described method.

Thus, the processing system 510 is configured to obtain head ultrasound imaging data of the target fetus, containing a representation of the brain and/or head of the target fetus; obtain a plurality of models, each model representing a structure of the brain and/or head of a fetus at a different gestational age; process the head ultrasound imaging data and the plurality of models to identify the model, amongst the plurality of models, that most closely fits to the structure of the brain and/or head of the target fetus; and generate a first prediction of the gestational age of the target fetus using the gestational age associated with the identified model.

The user interface 520 is configured to provide a user-perceptible output responsive to the first prediction. The processing system 510 may control the operation of the user interface. The user-perceptible output may be controlled responsive to the first prediction alone or other data elements derived using the first prediction (such as the SGA indicator and/or the combined prediction previously described).

It will be appreciated that the processing system 510 may be implemented in an ultrasound imaging system or ultrasound acquisition system. Thus, the system 500 may form part of an ultrasound imaging system.

It will be understood that disclosed methods are preferably computer-implemented methods. As such, there is also proposed the concept of a computer program comprising code means for implementing any described method when said program is run on a processing system, such as a computer. Thus, different portions, lines or blocks of code of a computer program according to an embodiment may be executed by a processing system or computer to perform any herein described method. In some alternative implementations, the functions noted in the block diagram(s) or flow chart(s) may occur out of the order noted in the Figs.. For example, two blocks shown in succession may, in fact, be executed substantially concurrently, or the blocks may sometimes be executed in the reverse order, depending upon the functionality involved.

Variations to the disclosed embodiments can be understood and effected by those skilled in the art in practicing the claimed invention, from a study of the drawings, the disclosure and the appended claims. In the claims, the word "comprising" does not exclude other elements or steps, and the indefinite article "a" or "an" does not exclude a plurality. A single processor or other unit may fulfill the functions of several items recited in the claims. The mere fact that certain measures are recited in mutually different dependent claims does not indicate that a combination of these measures cannot be used to advantage. If a computer program is discussed above, it may be stored/distributed on a suitable medium, such as an optical storage medium or a solid-state medium supplied together with or as part of other hardware, but may also be distributed in other forms, such as via the Internet or other wired or wireless telecommunication systems. If the term "adapted to" is used in the claims or description, it is noted the term "adapted to" is intended to be equivalent to the term "configured to". Any reference signs in the claims should not be construed as limiting the scope.

## Claims

1. A computer-implemented method (100, 300) of predicting a gestational age of a target fetus, the computer-implemented method comprising:
obtaining (110) head ultrasound imaging data (115) of the target fetus, containing a representation of the brain and/or head of the target fetus;
obtaining (120) a plurality of models (125, 210, 220, 230), each model representing a structure of the brain and/or head of a fetus at a different gestational age;
processing (130) the head ultrasound imaging data and the plurality of models to identify the model, amongst the plurality of models, that most closely fits to the structure of the brain and/or head of the target fetus; and
generating (140) a first prediction (145) of the gestational age of the target fetus using the gestational age associated with the identified model.

2. The computer-implemented method of claim 1, wherein the step of processing the head ultrasound imaging data and the plurality of models comprises:
segmenting the ultrasound imaging data to generate a segmentation mask representing the structure of the brain and/or head of the target fetus;
fitting the segmentation mask to each model of the plurality of models; and
identifying the model that most closely or best fits the segmentation mask.

3. The computer-implemented method of claim 1, wherein the step of processing the head ultrasound imaging data and the plurality of models comprises:
segmenting the ultrasound imaging data to generate a segmentation mask representing the structure of the brain and/or head of the target fetus;
comparing the segmentation mask and the part of the head ultrasound imaging data bound by the segmentation mask to each model of the plurality of models; and
identifying the model that most closely or best matches the segmentation mask and the part of the head ultrasound imaging data bound by the segmentation mask.

4. The computer-implemented method of claim 2 or 3, wherein the step of segmenting the ultrasound imaging data comprises:
processing the ultrasound imaging data to detect the presence or absence of one or more structural elements of the brain and/or head of the target fetus; and
segmenting the ultrasound imaging data to generate a segmentation mask that identifies the shape of only structural elements that are detected as present in the brain and/or head of the target fetus.

5. The computer-implemented method (300) of any of claims 1 to 4, further comprising:
obtaining (310) body ultrasound imaging data (315), containing a representation of a portion of the body of the target fetus;
processing (320) the body ultrasound imaging data to determine one or more anatomical measurements of the target fetus;
generating (325) a second prediction (329) of the gestational age of the target fetus by processing the determined anatomical measurements; and
generating (330) a small for gestational age, SGA, indicator (335) responsive to a difference between the first prediction and the second prediction, wherein the SGA indicator indicates a prediction of whether or not the target fetus is small for its gestational age.

6. The computer-implemented method of claim 5, further comprising a step (340) of providing a user-perceptible alert responsive to the SGA indicator meeting at least one predetermined criterion.

7. The computer-implemented method of claim 6, wherein the at least one predetermined criterion comprises the SGA indicator indicating a prediction that the target fetus is small for its gestational age.

8. The computer-implemented method of any of claims 5 to 7, wherein the SGA indicator indicates a prediction that the target fetus is small for its gestational age responsive to the difference between the first and second predictions exceeding a predetermined value.

9. The computer-implemented method of any of claims 5 to 8, further comprising determining (350) a combined estimation of the gestational age of the target fetus by processing the first prediction and the second prediction.

10. The computer-implemented method of any of claims 1 to 9, further comprising controlling (150) a user-perceptible output responsive to the first prediction.

11. The computer-implemented method of any of claims 1 to 10, wherein the structure of the brain and/or head of the target fetus includes the shape of one or more cerebral structures (215, 225, 235), such as sulci.

12. The computer-implemented method of any of claims 1 to 11, wherein the interval between consecutive gestational ages represented by the plurality of models is between one and two weeks.

13. A computer program product comprising computer program code means which, when executed on a computing device having a processing system, cause the processing system to perform all of the steps of the method according to any of claims 1 to 12.

14. A processing system (400, 510) configured to predict a gestational age of a target fetus, wherein the processing system is configured to:
obtain (110) head ultrasound imaging data (115) of the target fetus, containing a representation of the brain and/or head of the target fetus;
obtain (120) a plurality of models (125, 210, 220, 230), each model representing a structure of the brain and/or head of a fetus at a different gestational age;
process (130) the head ultrasound imaging data and the plurality of models to identify the model, amongst the plurality of models, that most closely fits to the structure of the brain and/or head of the target fetus; and
generate (140) a first prediction (145) of the gestational age of the target fetus using the gestational age associated with the identified model.

15. A system (500) comprising:
the processing system (510) of claim 14; and
a user interface (520) configured to provide (150) a user-perceptible output responsive to the first prediction.
